# EUROPEAN PATENT APPLICATION

(11) **EP 0 967 218 A1**
(43) Date of publication of application: **29.12.1999**
(21) Application number: 98201752.7
(22) Date of filing: 26.05.1998
(51) Int. Cl.: C07H 1/08

(54) **Silica-guanidiniumthiocyanate DNA isolation using casein**

(71) Applicant: ACADEMISCH ZIEKENHUIS BIJ DE UNIVERSITEIT VAN AMSTERDAM, 1105 AZ Amsterdam (NL)
(72) Inventor: The designation of the inventor has not yet been filed
(74) Representative: Smulders, Theodorus A.H.J., Ir.

(57) **Abstract**

DNA purified by the guanidiniumthiocyanate-silica procedure (Boom et al, J. Clin. Microbiol. 28:495-503, 1990) from some sources (e.g. cerebrospinal fluid and urine) has appeared to contain inhibitors of DNA processing enzymes (Taq DNA polymerase, restriction enzymes and DNA ligase). These inhibitors were, at least in part, introduced by the purification procedure itself. The inhibition of DNA processing enzymes could be relieved by the inclusion of alpha casein into the guanidiumthiocyanate (GuSCN) containing lysisbuffer. Alpha casein is copurified with DNA by simultaneous binding to the silica particles and is subsequently eluted together with the DNA in the last step of the purification procedure after which it exerts its beneficial effects for DNA processing enzymes.

## Description

The present invention relates to the field of molecular biology and biochemistry, in particular to the field of isolation, identification and/or processing of nucleic acids, such as RNA and DNA. It particularly relates to methods for isolating, identifying and/or processing of nucleic acids from biological samples. Typically the invention also relates to separating different kinds of nucleic acids from each other (DNA from RNA, single stranded nucleic acids from double stranded nucleic acids, etc.)

In our previous work we have provided the art with the so called guanidiniumthiocyanate-silica procedure, which when applied in different formats leads to isolation of different kinds of nucleic acids from all kinds of samples, in particular samples suspected to contain pathogens, such as viruses. Our work as referenced herein includes processing of nucleic acids obtained from these samples, for instance conduction restriction analysis, ligation of nucleic acids or amplification of specific nucleic acids obtained in the purification procedures. Nucleic acids purified by the guanidiniumthiocyanate-silica procedure (Boom et al, J. Clin. Microbiol. 28:495-503, 1990, incorporated herein by reference) from some sources (e.g cerebrospinal fluid and urine) has appeared to contain inhibitors of nucleic acid processing enzymes (Taq DNA polymerase, restriction enzymes and DNA ligase). These inhibitors apparently were, at least in part, introduced by the purification procedure itself. The present invention now provides a method and a means to suppress the inhibition of these enzymes. We have shown that the inhibition of processing enzymes can be relieved by the addition of (alpha) casein or a functional equivalent thereof to the process of isolation and/or purification of nucleic acids.

Thus the invention provides a method for isolating and identifying a target nucleic acid from samples containing biological material, comprising lysing said biological material with a lysis buffer and processing a target nucleic acid suspected to be present in said sample, whereby casein or a functional equivalent thereof is added to avoid inhibition of nucleic acid processing enzymes. As explained herein a target nucleic acid may be single stranded, double stranded, RNA or DNA or heteroduplexes or PNA, circular, linear, synthetic or natural. Basically this invention can be used to isolate, purify or separate nucleic acids of any origin or composition from any (aqueous) sample. It is of course preferred to use a method as disclosed in our earlier work, meaning that it is preferred to apply the methods to biological samples, in particular to isolate, purify, process and/or identify nucleic acids originating from pathogens, in particular viruses therein. Thus it is also preferred to use differential absorption to silica particles (or functional equivalents thereof) of different kinds of nucleic acids. It is of course also preferred to lyse the biological samples suspected to contain target nucleic acids using a lysis buffer, preferably said lysis buffer containing a chaotropic agent, which preferably is guanidiniumthiocyanate. Of course the present invention provides an improvement to all these methods when the nucleic acids purified or isolated or identified need to be processed. The addition of alpha casein or a functional equivalent thereof (whereby a functional equivalent is intended to read on similar proteins exerting a similar function, such as derivatives and/or fragments of alpha casein, or homologs of alpha casein, but also other suppressors of inhibitors of nucleic acid processing enzymes), in particular its addition before or during lysing prevents or at least suppresses the inhibition of the processing enzymes. It is particularly suitable to add casein or its equivalent to the lysis buffer, since it apparently copurifies with nucleic acids, in particular when a purification through absorption to silica particles is used, especially in combination with lysis by a chaotropic agent, in particular guanidiniumthiocyanate.

The casein or its equivalent can thus be suitably added to said lysis buffer.

It is preferred to apply the invention to methods whereby said processing includes amplification of target nucleic acid or methods wherein said processing includes restriction and/or ligation of target nucleic acids. The enzymes involved in these processes are those likely to be inhibited by inhibitors introduced by the purification process or present in the sample from the beginning.

As disclosed in the detailed description the invention has major advantages in yield and the like when applied to samples containing only few copies of the target nucleic acids. Thus in a preferred embodiment the invention provides a method whereby the target nucleic acids are present in the sample at the most at levels of 50.000 molecules per 200 µl of sample or less. Since the most important field of application of the invention is diagnosis and/or identification of pathogens (particularly viruses) in organisms, in particular in mammals the sample preferably is an aqeous body fluid, particularly derived from urine or cerebrospinal fluid and feces, in which kind of samples the methods according to the invention perform significantly better than the prior art methods.

### Detailed description.

### Summary.

As stated herein before nucleic acids and typically DNA purified by the guanidiniumthiocyanate-silica procedure (Boom et al, J. Clin. Microbiol. 28:495-503, 1990) from some sources (e.g cerebrospinal fluid and urine) has appeared to contain inhibitors of DNA processing enzymes (Taq DNA polymerase, restriction enzymes and DNA ligase). These inhibitors may, at least in part, have been introduced by the purification procedure itself. According to the invention the inhibition of nucleic acid (DNA) processing enzymes can be relieved by the inclusion of alpha casein into the guanidiniumthiocyanate (GuSCN) containing lysisbuffer. Alpha casein is copurified with DNA by simultaneous binding to the silica particles and is subsequently eluted together with the DNA in the last step of the purification procedure after which it exerts its beneficial effects for DNA processing enzymes.

We have compared a previously described lysisbuffer (L6) with a novel lysisbuffer (L7A), containing alpha casein, with respect to DNA yield and performance of DNA processing enzymes (Taq DNA polymerase, restriction enzymes and DNA ligase). The data show that the novel lysisbuffer performs equal to (for serum and plasma) or better (for water, urine, cerebrospinal fluid) than the original lysis buffer with respect to activity of DNA processing enzymes. Beneficial effects for Taq DNA polymerase were moderate (up to 10-fold increase in PCR product in a 35 cycle PCR) whereas effects for restriction enzymes were profound. Single-stranded (ss) DNA and double stranded (ds) RNA were isolated with similar efficiencies for both lysisbuffers, ss-RNA yields were, however, low relative to the original lysis buffer. Double-stranded DNA yields were the same (near 100%) for both lysisbuffers at high DNA levels (micrograms). At very low ds-DNA inputs (50 molecules to 40.000 molecules) yields were higher with the novel lysisbuffer relative to the original lysisbuffer as determined by quantitative PCR.

### Introduction.

It has been our main goal to develop reliable and sensitive diagnostic and quantitative assays based on the polymerase chain reaction (PCR, 35) for the detection and quantitation of herpesvirus DNA in clinical specimens like whole blood, serum, plasma and cerebrospinal fluid (CSF). In our laboratory, DNA from these specimens is routinely extracted by the silica-GuSCN procedure previously developed at our laboratory (4), subjected to PCR and amplimers are detected after liquid hybridisation with TBR {Tris (2,2'-bipyridine) ruthenium (II) chelate} labeled probes and electrochemiluminescence (ECL, QPCR system 5000, Perkin Elmer). Due to the presence of internal control DNA (35 molecules of a plasmid containing a DNA sequence mimicking viral target DNA) which is added together with the specimen as input for DNA extraction, the combined effects of DNA yield and the presence of PCR inhibitors can be monitored for, which is required for a reliable diagnostic assay. Although no inhibition was observed for whole blood, serum and plasma, low internal control signals were frequently obtained for e.g. CSF and urine. Such a result could be due to low extraction efficiency, inhibition of PCR, or both.

The silica-GuSCN nucleic acid (NA) purification method is based on the lysing and nuclease-inactivating properties of the chaotropic agent guanidiniumthiocyanate (GuSCN) together with the NA binding properties of silica particles in the presence of this agent. This procedure is now widely used for purification of both single-stranded (ss) and double-stranded (ds) DNA and RNA from clinical specimens in both qualitative and quantitative nucleic acid (NA) amplification assays for the detection of bacterial and viral pathogens in a variety of clinical specimens including serum, plasma, cerebrospinal fluid, ocular humour, urine, sputum, feces and tissue biopsies and has been successfully performed in the detection and quantitation of Human Immunodefiency Virus (45, 19, 39, 43, 33, 47, 44, 46), Hepatitis C virus (8, 26, 29), Hepatitis B Virus (5), Hepatitis E Virus (24), Parvovirus (28), (42), Bovine Polyoma Virus (38), Human Papilloma Virus (41), Human Cytomegalovirus (14, 49), Herpes simplex virus (31), Torovirus (21), Norwalk Virus (15), Enterovirus (50), Human Herpesvirus-6 (36), Rubella virus (32), Mycobacteria (7, 13, 20, 34, 37,), Leptospira (25), and Treponema (27). The NA isolation procedure has been acknowledged for its potency to remove inhibitory substances from clinical specimens (7, 48, 47, 32, 17, 13, 16, 12, 36, 49).

Herein we report that DNA purified from clinical specimens like CSF and urine frequently contained an inhibitor(s) of DNA processing enzymes (Taq DNA polymerase, restriction enzymes and DNA ligase) which was, at least in part, introduced by the purification procedure itself. Inhibition could be relieved by the addition of alpha-casein to the reaction mixtures or, preferably, by the use of a novel lysisbuffer (L7A) containing alpha casein. In the latter procedure, alpha casein was bound by the silica particles in the first step of the procedure and eluted together with DNA in the last step, after which it exerted its beneficial effects for DNA processing enzymes. The enhancement of restriction enzyme activity by casein present in the reaction mixture has been described previously (11). We have compared the previously described lysisbuffer (L6) with the novel lysisbuffer containing alpha casein (L7A) with respect to ds-DNA yield and the performance of DNA processing enzymes (Taq DNA polymerase, restriction enzymes and DNA ligase). In addition, some characteristics of the novel lysis buffer were studied for isolation of ds-RNA, ss-RNA and ss-DNA.

### Materials and methods

**Chemicals and enzymes.** Restriction enzymes, T4 DNA ligase, 48 kb phage Lambda DNA and phage M13 single-stranded DNA were from Boehringer Mannheim. Taq DNA polymerase (Amplitaq) was from Perkin Elmer. Alpha casein was obtained from Sigma Chemical Company (chromatographically purified to a purity of at least 85%, catalog nr. C 6780).

**Restriction enzyme analysis and agarose gel electrophoresis.** Restriction enzyme digestions of 1 µg of DNA were done for 1 hour at 37°C with 5U of enzyme in the buffer systems provided by the manufacturer. DNA was electrophoresed for 1-2 h through horizontal agarose slab gels (5 to 10 V/cm) in the buffer system described by Aaij and Borst (1) containing 1 µg ethidium bromide per ml and photographed under UV illumination.

**Polyacrylamide gel electrophoresis (PAGE).** Proteins were electrophoresed after reduction through 15% polyacrylamide-SDS gels as described by Laemmli (23) and were visualized by silver staining (6).

**Preparation of lysisbuffer L7A.** Lysisbuffer L7A was prepared by the addition of alpha casein to lysisbuffer L6 {prepared as described previously (4)} to a final concentration of 1 mg/ml L6. L7A was stable for at least 2 months when stored at room temperature in the dark. Concentrated (50x) stock solutions (50 mg alpha casein/ml L6) were stored at -20°C and were stable for at least one year.

**DNA purification.** DNA was purified from 200 µl CSF, serum, plasma, urine, water, or from 50 µl whole blood by protocol Y/SC (see Results section for outline) as described previously (4) with 20 µl of size-fractionated silica particles (SC) using either lysisbuffer L6 or lysis buffer L7A. DNA was eluted in 100 µl TE-buffer (10 mM Tris, 1 mM EDTA, pH=8,0).

**Virus.** Sucrose density gradient purified Human Cytomegalovirus (CMV) strain AD 169 {lot nr. 80-165-1, 5.38 x 10⁹ viral particles/ml virus dilution buffer (10 mM TRIS.HCl, 150 mM NaCl, 1 mM EDTA, pH 7.5), as determined electronmicroscopically by direct particle count which discriminated between full and empty particles} was obtained from Advanced Biotechnologies Inc (Columbia, MD, USA). According to the manufacturer the error in viral particle count was estimated to be ± 0.5 log. We therefore performed limiting dilution experiments of DNA purified by protocol Y/SC (4) from virus preparations followed by PCR; these experiments suggested that the actual amount of viral DNA was about 3 times higher than expected from the viral particle count assuming a 100% recovery of viral DNA (data not shown). Virus concentrations mentioned in this paper were based on this correction factor.

**PCR.** Primers (HPLC purified) and TBR {Tris (2,2'-bipyridine) ruthenium (II) chelate} labeled probes were from Perkin Elmer (PE); primers were diluted in TE buffer (10 mM TRIS.HCl, 1 mM EDTA, pH 8.0) to 100 µg/ml and stored at - 20°C; probes were diluted in lx PCRII buffer (PE, 10 mM TRIS.HCl pH 8.3, 50 mM KCl) to 1 µg/ml and stored at -20°C. The primer pair used for amplification consisted of CMV-A and Bio-CMV-B (5' biotinylated). This primer pair amplifies a 578 bp fragment from exon 4 (2) of the major immediate early gene of HCMV or a fragment of identical size and GC content from Internal Control DNA (see below). The final reaction mixture (50 pl) contained 28 pmoles of each primer CMV-A and Bio-CMV-B, 2.5 U of Amplitaq DNA polymerase, 0.5 U of Amperase (Uracil-N-glycosylase, Perkin Elmer), 5 mg BSA (Boehringer Mannheim), 10 mM TRIS-HCl (pH 8.3), 50 mM KCl, 3 mM MgCl₂, 200 mM each of dATP, dGTP and dCTP and 400 mM of dUTP (Perkin Elmer). PCR reactions were done in a Perkin Elmer 9600 thermocycler: 2 min at 50°C, 5 min at 95°C followed by 35 cycles each consisting of 20 sec at 95°C, 20 sec at 63°C, 1 min at 72°C; followed by 5 min at 72°C.

**Internal control DNA**. Internal control (IC) DNA was constructed (by standard procedures) such that a 25 bp DNA sequence present in a 578 bp CMV DNA fragment, bracketed by CMV-A and Bio-CMV-B, was replaced by a novel 25 bp DNA sequence to serve as a probe area. This modification allowed for discrimination between CMV and IC amplimers (which have the same size and GC content and are amplified by the same primer pair) resulting from the PCR described above, by probes specific for either CMV or internal control specific areas (see below). The modified DNA fragment (578 bp) was cloned into a plasmid vector (PCRII, 3932 bp, Promega) resulting in plasmid pCMV-marker. Details on the construction of pCMV-marker and on the nucleotide composition of primers and probes will be described elsewhere. pCMV-marker was purified from bacterial cultures as described (18) and the plasmid was linearized by HindIII digestion, purified by protocol Y/SC (4) and stored in TE buffer (at 100 µg plasmid/µl) at -20°C; very low concentrations (5-1000 molecules/µl) were made in TE buffer containing human chromosomal DNA (Sigma Chemicals) at 20 µg/ml TE buffer and stored at -20°C. The concentration of IC DNA was quantitated by UV absorption and by limiting dilution followed by PCR.

**Removal of excess primers.** Initial experiments revealed that the amount of biotin labelled primers used in the PCR reaction exceeded the biotin binding capacity of the streptavidin coated magnetic beads; therefore a protocol (Protocol Delta Y-A) was developed to remove excess primers from the PCR products. This protocol was based on the DNA binding of silica particles in the presence of the chaotropic agent guanidinium thiocyanate (4); details will be described elsewhere. DNA was eluted in 100 µl 1x PCRII buffer for 10 min at 56°C followed by vortexing and subsequent centrifugation for 2 min at approx. 12,000 x g. The supernatant contained the purified PCR product.

**Hybridisation and ECL measurement.** Purified PCR product was diluted 5 times in 1x PCR II buffer. To 30 µl of the diluted PCR product 20 µl of TBR labelled probe (1.3 pmoles, either CMV- or IC-specific) was added and hybridisation was done in a Perkin Elmer 9600 thermocycler (2 min at 95°C followed by 5 min at 56°C). Subsequently, 10 µl of streptavidin coated magnetic beads (Perkin Elmer) were added and the mixture was incubated for 15 min at 56°C. Forty µl of the beads-hybrids suspension were added to 400 µl QPCR assay buffer (Perkin Elmer) and the ECL signal, expressed in luminosity units, was measured in the QPCR system 5000 (Perkin Elmer). TBR labelled probes were TBR-CMV-1 (CMV-specific probe) and TBR-CMV-2 (IC-specific probe). Both probes contained a single TBR label at the 5' end. No cross hybridisation was observed for internal control and virus specific probes (data not shown).

**Quantitative PCR (QPCR).** DNA was purified from 200 µl plasma samples (supplemented with known amounts of CMV) with either L6 or L7A as lysis buffer and DNA was eluted in 100 µl TE buffer. Twenty µl of DNA were subjected to PCR in the presence of 35 molecules of IC DNA, and ECL signals obtained after hybridisation with TBR labeled probes were determined as described above. Background signals for virus-specific (mean 30 lum. units) and IC-specific probes (mean 24 lum. units) were determined for 23 plasmas previously shown to be CMV negative. After background correction, the ratio of Virus-specific signal/IC-specific signal (R) was calculated and the copy number of CMV per ml plasma was calculated by amplifying R with a factor 875. This factor was reached from separate factors 35 x 5 x 5, which represent the number of IC DNA molecules present during the PCR, a correction factor for the amount of DNA used in the PCR reaction (20% of the DNA extracted from 200 µl plasma), and a correction factor to reach the copy number per ml plasma, respectively. This algorithm assumes ideal circumstances for each of the steps of the procedure and would be valid if 1) recovery of viral DNA from plasma were 100% and, 2) if viral and Internal Control DNA were amplified with equal efficiencies, 3) if virus and IC amplimers were purified with equal efficiencies and, 4) if hybridisation with either probe were quantitative and, 5) if measurement -by ECL- of the amount of hybrids were quantitative. Experimental results concerning these considerations and the validation of the CMV QPCR will be discussed elsewhere (Boom et al. in preparation).

Reconstruction experiments showed that extraction efficiency with lysis buffer L7A at extremely low levels of input DNA (4 molecules of internal control DNA/200 µl plasma), was near 100% (data not shown).

### Results

In the first step of the silica-GuSCN procedure (4), a clinical specimen is added to a mixture of silica particles and a lysis buffer with a high concentration (5.25M) of the chaotropic agent GuSCN (lysis buffer L6). In this mixture, rapid lysis of viruses, cells and Gram negative bacteria occurs, together with inactivation of DNAses and RNAses. DNA and RNA released from lysed target organisms is bound by the silica particles. The NA-silica complexes are then washed twice with a buffer with a high GuSCN content (wash buffer L2), twice with 70% ethanol and once with acetone, the complexes are dried and DNA and RNA is eluted in TE buffer. Using this basic procedure (with lysis buffer L6) we observed inhibition of DNA processing enzymes for DNA purified from CSF and urine whereas DNA purified from serum, plasma (irrespective of the anticoagulants used) and whole blood did not show inhibition. Initial experiments showed that the inhibitor(s) was introduced, already bound to the silica particles, in the first step of the procedure, remained bound throughout the washing steps and was eluted from the silica particles in the last step of the procedure. Although the inhibitor(s) could be removed from silica particles by extensive washing of the particles at elevated temperatures (10 mM EDTA, pH 8.0 at 56°C), we have not yet succeeded in the preparation of bulk amounts of size fractionated silica particles which are free of the inhibitor (R. Boom, unpublished data).

In this paper we have compared the performance of the original lysisbuffer L6 with a novel lysis buffer, L7A (simply prepared from lysisbuffer L6 by addition of alpha casein). L6 eluate and L7A eluate resulted from extractions with lysis buffer L6 and L7A respectively. To monitor for inhibitors of restriction enzymes present in these eluates we performed extractions with different input materials (pure water, serum, plasma, urine, CSF). Subsequently a constant amount of previously purified DNA (commercially available 48 kb phage lambda DNA) was added to the eluates and the activity of restriction enzymes was determined. This type of experiments allowed conclusions on the presence of inhibitors of restriction enzymes.

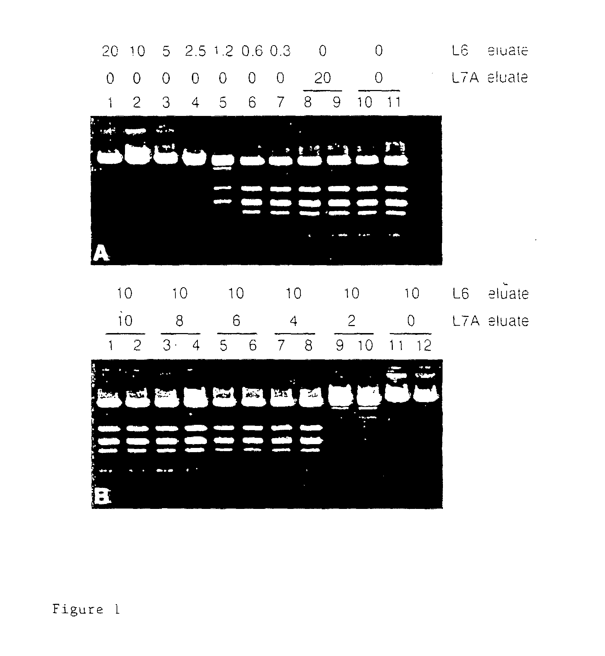

**Inhibition of EndoR activity by L6 eluate and relief of inhibition by L7A eluate.** In Figure 1A (lanes 1-7) it is shown that an inhibitor of EcoRI was present in L6 eluate resulting from an extraction of pure water since dilution of the eluate restored EcoRI activity. This experiment showed that inhibitors were introduced by the DNA extraction procedure itself. In Fig 1A (lanes 8 and 9) it can be seen that EcoRI inhibition was not observed when extractions were done when lysisbuffer L6 was replaced by lysis buffer L7A, containing alpha casein. Apparently the inhibitor was no longer present in the L7A eluate or alternatively, the inhibitor in L6 eluate was neutralized by a factor present in the L7A eluate. The data presented in Figure 1B (lanes 1-10) show that the addition of L7A eluate to L6 eluate relieved EcoRI inhibition suggesting that the inhibitor in the L6 eluate was neutralized by a factor (alpha casein, as shown below) present in the L7A eluate.

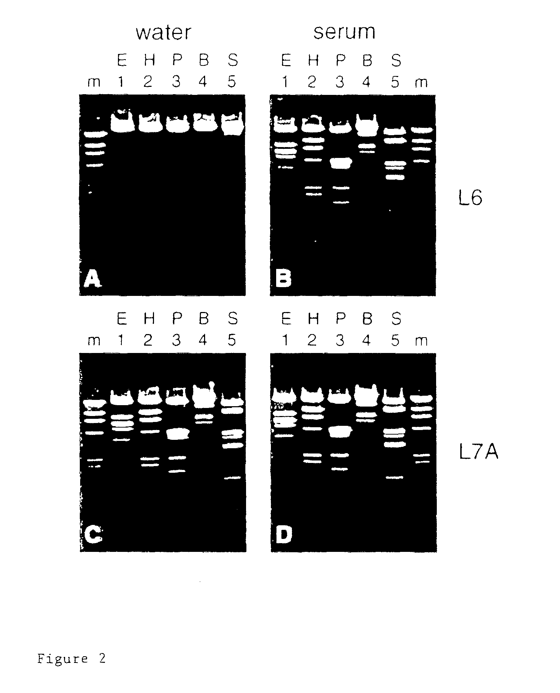

Figures 2A and 2C show that the beneficial effect of lysisbuffer L7A was not restricted to EcoRI activity but was also observed for other restriction enzymes when L6 eluates resulting from a water extraction were tested. In similar experiments inhibitors in L6 eluates were also observed for other restriction enzymes like HinfI, KpnI, SphI, CfoI, HaeIII, and HindII, whereas no inhibition was found for PstI, MspI, HpaII and HhaI; no inhibition of restriction enzymes was found in L7A eluates (not shown). Inhibition by L6 eluates was not restricted to phage lambda DNA but was also observed for other DNA sources like plasmids purified by a standard (18) procedure (not shown).

A completely different situation was encountered when serum, rather than water, was used as input material for extraction since no inhibition of restriction enzymes was observed using either lysisbuffer (Figures 2B and 2D). This phenomenon was not restricted to a particular serum since 10 other sera gave similar results (not shown). Apparently when serum was used as input for extraction the inhibitor was not present or, alternatively, a factor was copurified from serum which relieved inhibition. To discriminate between these possibilities, a fixed amount of L6 eluate from a water extraction which resulted in partial digestion of template DNA was supplemented with increasing amounts of L6 eluate resulting from a serum extraction. In this way the presence of inhibitors as well as the presence of enhancers of restriction enzyme activity could be monitored since the pattern of the partial digestion products would change in either case. Thus, if inhibitors were present in the serum eluate the partial digestion pattern would shift to a pattern corresponding to a lower enzyme activity, on the other hand when enhancers of restriction enzymes were present in the serum eluate this would result in a digestion pattern corresponding to a higher enzyme activity. Several experiments like those outlined above have suggested that L6 eluates resulting from serum extractions were indifferent i.e. these did not contain inhibitors nor components which could relieve inhibition (data not shown).

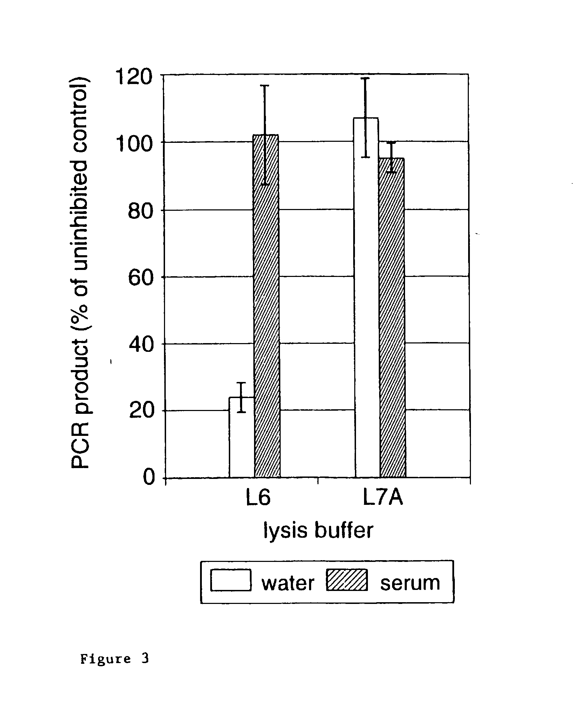

To monitor for inhibitors of Taq DNA polymerase, a constant amount of template DNA was added to L6 and L7A eluates and PCR reactions were done after which the amount of PCR product was determined by hybridisation with TBR-labelled probes and electrochemiluminescence. This type of experiment allowed conclusions on PCR inhibitors present in the eluates regardless of DNA yields (10). Fifty molecules of template DNA were added to the same eluates used in the experiment described in Figure 2, subjected to PCR and the amount of PCR product was determined. The primer pair amplifies a 578 bp fragment from exon 4 of the major immediate early gene of HCMV and a fragment of the same size and overall base composition from IC DNA. In Figure 3 it can be seen that the results obtained for PCR followed those obtained for restriction enzymes. Inhibition of PCR (to levels of 24% of uninhibited controls) was observed for the L6 eluate resulting from water extraction, inhibition was not observed for the L7A eluate. Inhibition of the L6 eluate could be relieved by dilution with TE buffer (not shown). On the other hand, when extraction was done with serum as input for extraction, no inhibition was found regardless of the lysisbuffer used. Similar results were obtained when the amounts of PCR products were examined by agarose gel electrophoresis and ethidium bromide staining (not shown).

Together the data presented above suggest that when lysisbuffer L6 was used for DNA extraction, an inhibitor of Taq DNA polymerase and restriction enzymes may be introduced by the DNA extraction procedure itself; when lysis buffer L7A was used, a factor (alpha casein as discussed below) was copurified which relieved inhibition.

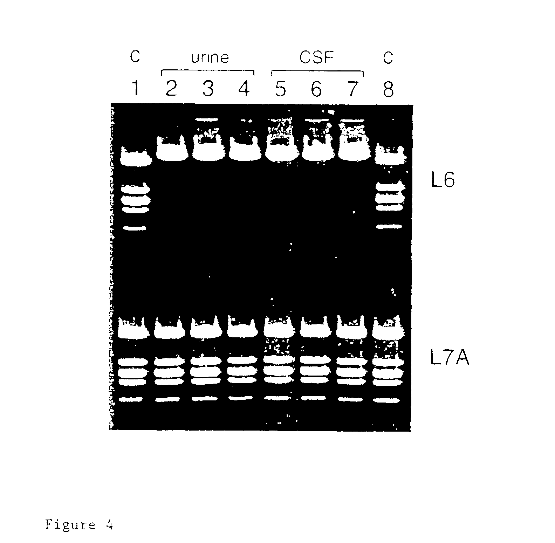

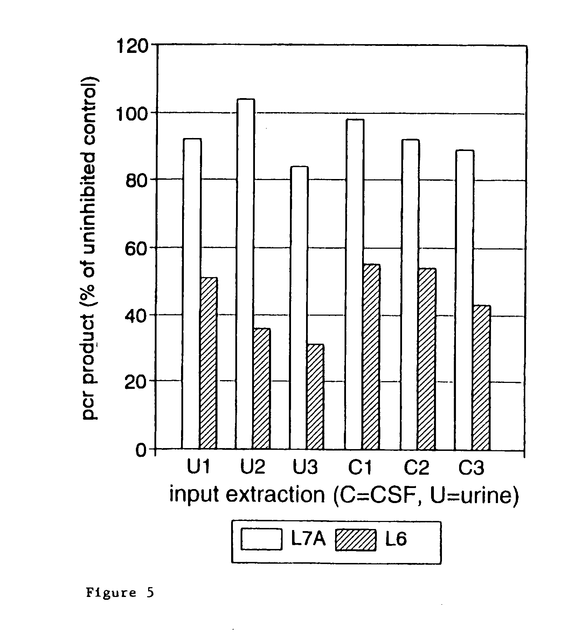

Although no significant inhibition of EndoRs and Taq DNA polymerase (and T4 DNA ligase, not shown) was found when the extraction was performed on serum or plasma by the standard procedure with L6 as lysis buffer, other clinical materials like urine or CSF behaved like water, that is, L6 eluates showed inhibition of restriction enzymes whereas no inhibition was observed in L7A eluates (as shown for 3 CSF specimens and 3 urine specimens in Figure 4). Similarly, PCR efficiency tested for the same specimens in the same experiment showed a moderate increase (two to 3-fold) in the amount of PCR product when lysis buffer L6 was replaced by L7A (Figure 5). Other experiments have shown that up to 10-fold increases in PCR products (in a 35-cycle PCR) could be obtained.

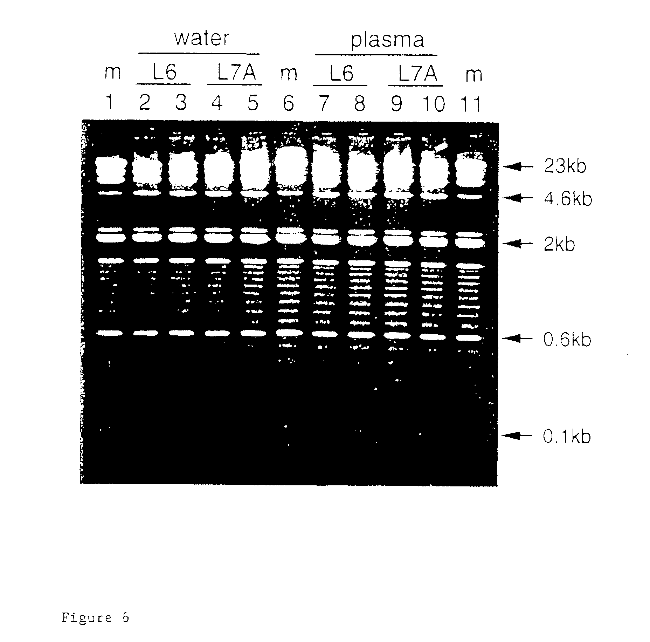

To establish the performance of the novel lysisbuffer with respect to DNA yield at relatively high DNA inputs (µg level), plasma or water was mixed with a constant amount of double-stranded DNA (a mixture of a HindIII digest of phage lambda DNA and a 100 bp DNA ladder). DNA was subsequently extracted from these mixtures using either L6 or L7A as lysisbuffer. In Figure 6 it can be seen that yields were the same for both lysisbuffers in the range of 23 kb to 100 bp; visual comparison with the 100% recovery markers (Figure 6, lanes 1, 6 and 11) suggested that ds-DNA yields were near 100%.

When target DNA is purified from clinical specimens and subjected to PCR, the amount of PCR product is not necessarily a direct reflection of the amount of target DNA present in the specimens since both DNA yield and PCR efficiency (influenced by inhibitors) will determine the amount of PCR product. In the PCR experiments described above, the presence of inhibitors was analyzed by adding a fixed amount of template DNA to L6 eluates and L7A eluates; these experiments were necessary to uncouple DNA yield and PCR efficiency but did not provide information about DNA yields obtained by the extraction procedure.

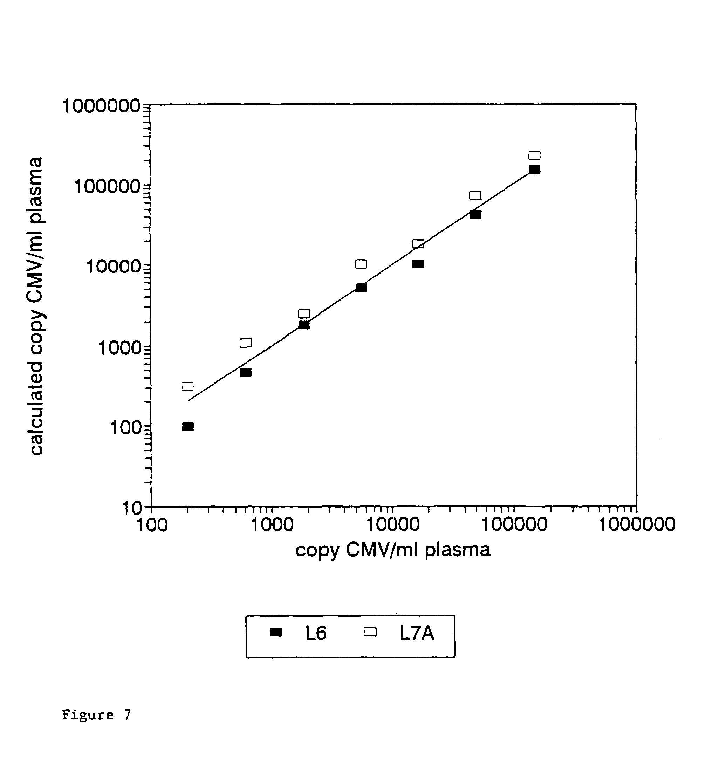

To study ds-DNA yield quantitatively at low DNA inputs, a human CMV-negative plasma was supplemented with a known amount of human Cytomegalovirus and serial threefold dilutions were made in the same plasma. DNA was purified from 200µl plasma samples and was subjected to a quantitative PCR recently developed in our laboratory (R. Boom et al, manuscript in preparation). Since quantitation of viral DNA is based on the presence of a fixed amount of IC DNA during PCR, DNA yield can be studied irrespective of the presence of PCR inhibitors. The data presented in Figure 7 suggest that ds-DNA yields obtained with the novel lysis buffer were slightly higher than those obtained with the original lysis buffer in the range of about 40 to 30,000 molecules of virus DNA per 200 µl plasma. Absolute yields for IC DNA purified from plasma with lysisbuffer L7A was near 100% at an input of 4 molecules per 200 µl plasma (as determined by limiting dilution PCR; data not shown).

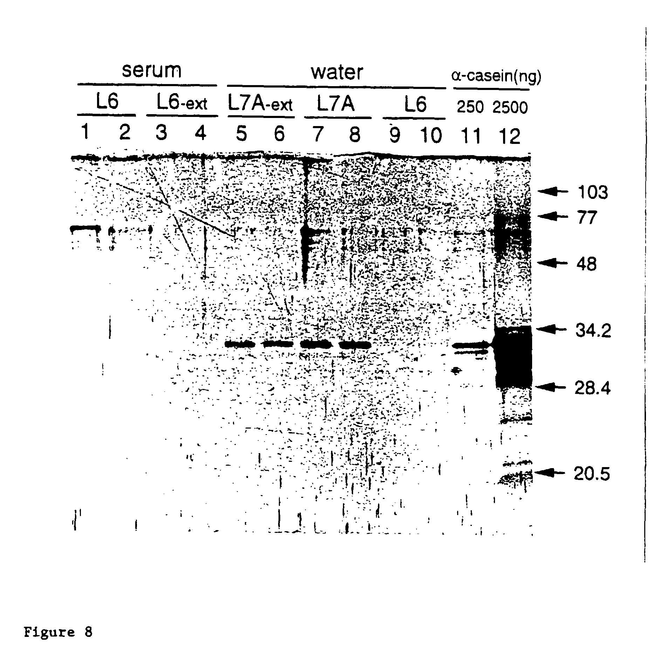

**Alpha casein is bound by silica particles and elutes in the last step of the procedure.** The data presented in figure 1 suggested that, when lysis buffer L7A was used, a factor was present in the eluate which could relieve inhibition of restriction enzymes. Relief of inhibition was still observed when extractions were done in which the silica pellet was washed 5 (rather than 2) times with wash buffer L2 , 5 (rather than 2) times with ethanol and 5 (rather than 1) times with acetone and, in addition, the silica suspension was transferred to a new tube after each wash step. Since alpha casein readily dissolves in wash buffer L2 (which is about 5M in GuSCN) the five wash steps with wash buffer L2 resulted in a dilution of alpha casein with at least a factor 7x10⁸, assuming a 30-fold dilution for each wash step (including the lysis step in L7A). Additional experiments showed that the amount of alpha casein that might be still present in the void volume of the silica pellets after these wash steps could not account for the observed relief of inhibition (data not shown). Together these experiments suggested that the factor was bound to the silica particles rather than to the wall of the reaction tube. Since this factor most likely was a protein, we did extractions using lysis buffer L6 or L7A, with either water or serum as input and studied the eluates for the presence of proteins by PAGE and silver staining. From Figure 8 it is obvious that when lysis buffer L7A was used for extraction (Fig. 8, lanes 7 and 8) a protein was purified (of approximately 32 kDa) which comigrated with alpha casein (Fig. 8, lanes 11 and 12). Since this result would also have been obtained when alpha casein was bound to the walls of the reaction tubes, we performed additional extractions in which the silica pellet was extensively washed (5 times) with wash buffer L2 and, in addition, the silica particle-L2 suspension was transferred to a new reaction tube after each of these wash steps. The data show that alpha casein was still recovered (Figure 8, lanes 5 and 6). In contrast, no proteins were observed in the L6 eluate when serum, which is very rich in proteins, was used as input for extraction (Figure 8, lanes 1-4) suggesting that protein binding was not a general property of the silica particles.

### Discussion

Herein we have compared the performance of the previously described lysis buffer L6 (4) with a novel lysis buffer L7A (prepared from lysisbuffer L6 by the addition of alpha casein) in the Silica-GuSCN DNA extraction procedure with respect to the presence of inhibitors of DNA processing enzymes (restriction enzymes, Taq DNA polymerase and DNA ligase) and DNA extraction efficiency for clinical specimens like serum, urine and CFS. We have shown that, when pure water was used as input material in the silica-GuSCN DNA extraction procedure in which the original lysis buffer L6 is used, an inhibitor(s) of DNA processing enzymes was present in the eluate which was apparently introduced by the purification procedure itself (Figure 1A). A similar inhibitory effect was observed when CSF and urine were used as input material for extraction (Figures 4 and 5). In contrast, no inhibition was found for serum (Figures 2 and 3), plasma (irrespective of the anticoagulants) and whole blood (not shown). Whereas inhibition was moderate for Taq DNA polymerase (up to 10 fold increase in the amount of PCR products in a 35 cycle PCR; Figures 3 and 5) and DNA ligase (not shown), strong inhibition was observed for several (but not all) restriction enzymes (Figures 2 and 4). The nature of the inhibitor(s) is not known but additional experiments have suggested that the inhibitor originated from the size-fractionated silica particle suspension (4) and appeared to be tightly bound to the silica particles. The inhibitor remained bound throughout the purification procedure and was eluted, at least in part, from the silica particles in the last step of the procedure. The inhibitor(s) could not be removed from silica particles (prior to extraction) by extensive washing of the particles with water but could be removed by extensive washing with 10 mM EDTA (pH 8.0). When inhibitor-free silica particles were used in the extraction procedure, no inhibitors were detected in the eluates showing that the extraction procedure per se did not introduce inhibitors (data not shown).

We showed that inhibition could be relieved by the inclusion of alpha casein into the reaction mixtures of PCR, restriction enzyme digestions and DNA ligase reactions (100 pg alpha casein/ml reaction mixture; not shown). These data extend the earlier observation of Dreyer and Schulte-Holthausen (11) that casein enhanced restriction enzyme activity. In their paper, the enhancing effect was not discussed in terms of inhibitors in general, nor to inhibitors introduced by a particular DNA isolation procedure but in terms of enhancement of restriction enzyme activity and was speculated to be due to allosteric changes (11). Inhibition could also be relieved by using a novel lysis buffer, L7A, which was simply prepared from the original lysisbuffer L6 by the addition of alpha casein. In this case alpha casein was bound to the silica particles in the first step of the DNA extraction procedure, remained bound throughout the washing steps and co-eluted with DNA, exerting its beneficial effects in subsequent enzymatic reactions. We preferred to add alpha casein to the lysisbuffer rather than to the reaction mixtures for practical reasons since only one buffer (L6) had to be changed. In addition, alpha casein as supplied by Sigma (chromatographically purified from bovine milk to a purity of 85%) appeared to be a mixture of several proteins (Figure 8). After extraction with silica only one, and occasionally a minor protein band migrating just ahead of the major band, was visible after PAGE and silver staining. Apparently the active component present in the casein preparation was selectively isolated from the mixture of proteins comprising the alpha casein preparation.

In contrast to CSF, urine and water, no inhibition was found using the original lysis buffer L6 when clinical specimens like serum, plasma and whole blood were used as input material for DNA extraction (Figures 2 and 3). If inhibitors were introduced by the extraction procedure, why did this not result in inhibition in these cases as well? One reason might be that components of blood, serum and plasma prevented copurification of the inhibitor. Alternatively, factors relieving inhibition like serum albumin, which is a known scavenger of PCR inhibitors (22), might be copurified from the clinical specimen. We have extensively tested the latter possibility but we have not found evidence that this was the case. Thus, experiments not shown here, have suggested that L6 eluates resulting from serum extractions did not contain inhibitors, nor components which could relieve inhibition. However, as soon as these samples not containing inhibitors are modified in any manner, the same problems in processing may appear. Of course the inclusion of casein or an equivalent thereof in the methods to isolate or purify, etc., will then take care of these problems. In general, in order to avoid these problems it is best to add alpha casein or an equivalent in these methods anyway, no matter what the original sample is.

The novel lysis buffer L7A was also examined with respect to DNA and RNA yields relative to the original lysis buffer L6. When tested for inputs in the microgram range, ds-DNA (100-10,000 bp) was isolated with the same yields (near 100%) for either lysis buffer (Figure 6). Recovery of very low amounts of high molecular weight ds-DNA (CMV DNA, 237 kb) was studied by quantitative PCR using an internal quantitation standard. The data (Figure 7) suggested that yields were higher with the novel lysis buffer L7A relative to the original lysis buffer L6, at extraction inputs ranging from about 40 to 30,000 molecules. Absolute yields for IC DNA (4,5 kb linear ds-DNA) purified from plasma with lysisbuffer L7A were about 100% at an extraction input of 200 ml plasma supplemented with only 4 molecules of IC-DNA (Boom et al., in preparation). At the microgram level, ss-DNA (phage M13 DNA) yields were slightly higher (estimated -from ethidium bromide stained gels- 50%) for the novel lysis buffer L7A than for the original lysis buffer L6 (estimated 30%, data not shown).

The basic NA isolation procedure with lysis buffer L6 and silica particles (4) has been used by many investigators for the isolation of low amounts of single-stranded RNA from e.g. Human Immunodefiency Virus (45, 19, 39, 43, 33, 47, 44, 46) and Hepatitis C virus (8, 26, 29). Initial experiments with ss-RNA inputs have suggested that the novel lysis buffer L7A is not as suitable for ss-RNA extraction due to low (less than 10%) ss-RNA yields. This low ss-RNA yield was not attributable to nuclease activity since L7A eluates have appeared to be devoid of both DNAses and RNAses (not shown). However, optimization of the method will probably result in good results for ssRNA as well. Double-stranded RNA on the other hand was recovered (from feces specimens containing human Rotavirus) with the same efficiency for either lysis buffer with estimated absolute yields of 60-70% (by visual comparison of ds-RNA inputs and outputs in ethidium bromide stained agarose gels; data not shown).

A modified lysis buffer may not only affect NA yields but may also affect lysis of the target organism in the first step of the procedure; at present we have no indication that there are gross differences in the lysing properties of lysis buffer L6 and L7A for the viruses studied thus far (human Cytomegalovirus, Herpes simplex virus, Adenovirus and human Rotavirus).

We were surprised to find that, in addition to nucleic acids, alpha casein was bound by silica particles in the presence of a high concentration of the chaotropic agent guanidiniumthiocyanate. To our knowledge this is the first time that the copurification of a protein (alpha casein) by the silica-GuSCN procedure is described. Protein binding was apparently not a general property of silica particles since no serum proteins were isolated and, in addition, alpha casein {which has a reported molecular weight of approximately 32 kDa (30)} was selectively isolated from the mixture of proteins comprising the alpha casein preparation (Figure 8).

It may be speculated that the binding of alpha casein to silica particles is mediated by phosphorylated serine residues which are abundantly present in alpha casein. Especially, stretches of phosphorylated serine residues (in the motif SerP-SerP-SerP-Glu-Glu) present in bovine alpha casein (40) might mimic DNA and might mediate binding by phosphate groups to silica particles. These phosphorylated regions give alpha casein a high chelating power towards positively charged calcium, magnesium, copper and iron ions (3, 9, 40) and might well be involved in the enhancing properties of alpha casein described here by capturing metal ions which otherwise might act as inhibitors of DNA processing enzymes. Alternatively, alpha casein might induce conformational changes of DNA and/or the DNA processing enzyme as suggested earlier (11).

In summary, we have compared the performance of a novel lysis buffer (L7A) containing alpha casein with the performance of the lysis buffer (L6) originally described in the silica- GuSCN nucleic acid purification procedure (4). Alpha casein copurified with NA by binding to silica particles and relieved the inhibition of DNA processing enzymes (Taq DNA polymerase, restriction enzymes and DNA ligase) that was observed when DNA was extracted from e.g. urine, CSF and water when the original lysis buffer (L6) was used. No inhibition was observed for either lysis buffer after DNA purification from serum, plasma and whole blood. Double-stranded DNA was isolated with high efficiencies from the microgram level to the level of a few (about 50) molecules for either lysis buffer. At the microgram level, ss-DNA and ds-RNA were recovered with efficiencies greater than 50% for the novel lysis buffer. However, ss-RNA was only recovered with very low yields in the case of lysis buffer L7A. No differences in lysing properties of lysis buffers L6 and L7A were observed for viruses. In addition to the data shown in this paper, beneficial effects of the novel lysis buffer were also observed when ds-DNA was purified from sources like feces, agarose blocks, reaction mixtures of DNA processing reactions (e.g. restriction enzyme digests, PCR products), and the like.

### Figure legends

### Figure 1. Inhibition of EndoR activity by L6 eluate and relief of inhibition by L7A eluate.

Extractions were done by protocol Y/SC with either lysis buffer L6 or lysisbuffer L7A with 200 µl water as input for extraction, and silica pellets were eluted with 100 µl TE buffer. Phage lambda DNA (1 µg, 48 kb) was added to these eluates and mixtures thereof, and DNA was cleaved by EcoRI (5U) in a 30 µl reaction volume (by adjustment with TE buffer) for 1 h at 37°C. Digests were electrophoresed through a 1% agarose gel and photographed under UV illumination.
(a). Decreasing amounts of L6 eluate (20, 10, 5, 2.5, 1.25, 0.6 and 0.3 µl, lanes 1-7 respectively). Control digestions: lanes 8 and 9, 20 µl L7A eluate; lanes 10 and 11, TE buffer only.
(b). To a constant amount of L6 eluate (10 µl), decreasing amounts of L7A eluate were added. Lanes 1 and 2, 10 µl L7A eluate; lanes 3 and 4, 8 µl L7A eluate; lanes 5 and 6, 6 µl L7A eluate; lanes 7 and 8, 4 µl L7A eluate; lanes 9 and 10, 2 µl L7A eluate, lanes 11 and 12, no L7A eluate.

### Figure 2. Absence of inhibition of restriction enzymes after serum extraction, relief of inhibition after water extraction by lysis buffer L7A.

Extractions were done by protocol Y/SC with either lysis buffer L6 (panels A and B) or lysisbuffer L7A (panels C and D) with either 200 µl water (panels A and C) or 200 µl serum (panels B and D) as input for extraction, and the resulting silica pellets were eluted with 100 µl TE buffer. Next, phage lambda DNA (1 pg, 48 kb) was added to 20 µl of the eluates and DNA was cleaved by different restriction enzymes (5U) in a 30 µl reaction volume for 1 h at 37°C. Lanes 1, EcoRI; lanes 2, HindIII; lanes 3, PvuII; lanes 4, BamHI; lanes 5, SspI, lanes m, length marker, HindIII digest of phage lambda DNA (500 ng). Digests were electrophoresed through a 1% agarose gel and photographed under UV illumination.

### Figure 3. Absence of inhibition of PCR after serum extraction, relief of inhibition after water extraction by lysis buffer L7A.

Extractions were done by protocol Y/SC with either lysis buffer L6 or lysisbuffer L7A with either 200 µl pure water or 200 µl serum as input for extraction, and the resulting silica pellets were eluted with 100 µl TE buffer. Fifty molecules of internal control DNA were added to 20 µl of the eluates and subjected to a 35 cycle PCR. The amount of PCR product was determined by hybridisation with a TBR-labeled probe specific for IC DNA and ECL. The amount of PCR product was expressed as percentage of uninhibited control reactions which contained 20 µl of TE buffer rather than L6 or L7A eluate. Open boxes, water input for extraction; hatched boxes serum input for extraction. PCR reactions were done in fourfold, means ± standard deviations are given. The eluates used in this experiment were obtained from the experiment described in Figure 2.

### Figure 4. Inhibition of restriction enzymes after extraction from urine and CSF is not observed with lysisbuffer L7A.

Extractions were done by protocol Y/SC with either lysis buffer L6 or lysisbuffer L7A with 200 µl each of 3 different urine specimens or 200 µl each of 3 different CSF specimens as input for extraction, and the resulting silica pellets were eluted with 100 µl TE buffer. Next, phage lambda DNA (1 µg, 48 kb) was added to the eluates and DNA was digested by EcoRI (5U) in a 30 µl reaction volume for 1 h at 37°C. Digests were electrophoresed through a 1% agarose gel and photographed under UV illumination. Top panel, lysis buffer L6, lower panel, lysisbuffer L7A. Lanes 1 and 8, control digests in TE buffer, lanes 2-4 urine specimens; lanes 5-7, CSF specimens.

### Figure 5. Inhibition of PCR after extraction from urine and CSF is not observed with lysisbuffer L7A.

Extractions were done with either lysis buffer L6 or lysisbuffer L7A with 200 µl each of 3 different urine specimens or 200 µl each of 3 different CSF specimens as input for extraction, and the resulting silica pellets were eluted with 100 µl TE buffer. Fifty molecules of IC DNA were added to 20 µl of the eluates and subjected to a 35 cycle PCR. The amount of PCR product was determined by hybridisation with a TBR labelled probe specific for IC DNA and ECL. The amount of PCR product was expressed as percentage of uninhibited control reactions which contained TE buffer rather than L6 or L7A eluate. Open boxes, L7A eluates; hatched boxes, L6 eluates. The eluates used in this experiment were the same as those used in the experiment described in Figure 4.

### Figure 6. Recovery of double-stranded DNA.

A mixture of HindIII digested phage lambda DNA (4pg) and 100 bp ladder (2 µg) was added to 100 µl water or to 100µl plasma. DNA was extracted from these mixtures using either L6 or L7A as lysisbuffer. DNA was eluted in 100 µl TE buffer and 25 µl were electrophoresed through a 1.5 % agarose gel and photographed under UV illumination. Lanes 1, 6 and 11, 100% recovery markers (m); lanes 2-5 extraction from water: lanes 2 and 3, with L6 as lysis buffer; lanes 4 and 5, with L7A as lysis buffer; lanes 7-10, extraction from plasma: lanes 7 and 8, L6 as lysis buffer; lanes 9 and 10, L7A as lysis buffer. DNA fragment lengths are indicated in kb; the apparent higher recovery of the 4,6 kb phage lambda DNA fragment in comparison to the marker lanes is due to the elution step at 56°C which causes separation of the cohesive ends of phage lambda DNA which link the 23 kb and 4.6 kb fragments.

### Figure 7. Recovery of low amounts of Cytomegalovirus DNA from plasma as determined by QPCR.

Human cytomegalovirus was added to a human plasma to a concentration of 150,000 virions/ml and the mixture was serially 3-fold diluted in the same plasma (lowest concentration 206 virions/ml plasma). DNA was extracted from 200 µl of these plasma samples with either lysis buffer L6 or lysis buffer L7A, and subjected to QPCR in the presence of a fixed amount of IC DNA. The amount of virus DNA was calculated from the ratio of virus-specific ECL signals over IC-specific ECL signals as described in the Methods section. Negative controls (plasma not supplemented with virus) were included and were found to be negative (not shown). Filled squares, L7A; open squares, L6; line, reference line which would be obtained when the calculated copy number/ml plasma and the true copy number/ml plasma were the same.

### Figure 8. Alpha casein is bound by silica particles.

Extractions were done (in duplicate) with 200 µl serum (lanes 1-4) or 200 µl water (lanes 5-10) as input for extraction. Either lysis buffer L6 (lanes 1-4, 9 and 10) or lysis buffer L7A (lanes 5-8) was used in the standard extraction protocol with 2 washes with wash buffer L2. In addition, extractions were done in which the silica particles were extensively (Ext) washed (five washes with washing buffer L2) and the silica particle/L2 suspension was transferred to a new eppendorf tube after each of these wash steps (lanes 3-6). Elution was with 60 µl TE buffer, 10 µl was electrophoresed through a 15% polyacrylamide gel followed by silver staining. Marker lanes contained 250 ng (lane 11) and 2500 ng (lane 12) alpha casein. Molecular weights (in kDa) are indicated.

### REFERENCES

1. **Aaij, C., and P. Borst.** 1972. The gel electrophoresis of DNA. Biochim. Biophys. Acta **269:**192-200.
2. **Akrigg, A., G.W.G. Wilkinson and J.D. Oram.** 1985. The structure of the major immediate early gene of human cytomegalovirus strain AD169. Virus Research **2:**107-121.
3. **Bernos, E., J.M. Girardet, G. Humbert, and G. Linden.** 1997. Role of the O-phosphoserine clusters in the interaction of the bovine milk alpha sl-, beta-, kappacaseins and the PP3 component with immobilized iron (III) ions. Biochim. Biophys. Acta **1337:**149-159.
4. **Boom, R., C.J.A. Sol, M.M.M. Salimans, C.L. Jansen, P.M.E. Wertheim-van Dillen and J. van der Noordaa.** 1990. Rapid and simple method for purification of nucleic acids. J. Clin. Microbiol. **28:**495-503.
5. **Boom, R., C.J.A. Sol, R. Heijtink, P.M.E. Wertheim-van Dillen and J. van der Noordaa.** 1991. Rapid purification of Hepatitis B Virus DNA from serum. J. Clin. Microbiol. **29:**1804-1811.
6. **Blum, H., H. Beier, and H.J. Gross.** 1987. Improved silver staining of plant proteins, RNA and DNA in polyacrylamide gels. Electrophoresis **8:**93-98, 1987
7. **Brisson-Noel A., C. Aznar, C. Chureau, S. Nguyen, C. Pierre, M. Bartoli, R. Bonete, G. Pialoux, B. Gicquel, and G. Garrigue.** 1991. Diagnosis of tuberculosis by DNA amplification in clinical practice evaluation. Lancet **338:**364-366.
8. **Cheung R.C., S.M. Matsui, and H. Greenberg.** 1994. Rapid and sensitive method for detection of hepatitis C virus RNA by using silica particles. J. Clin. Microbiol. **32:**2593-2597.
9. **Chruscinska, E., M. Dyba, G. Micera, W. Ambroziak, J. Olczak, J. Zabrocki, and H. Kozlowski.** 1997. Binding ability of Cu²⁺ ions by opiate-like fragments of bovine casein. J. Inorganic Biochem. **66:**19-22.
10. **Cone, R.W., A.C. Hobson, and M.W. Huang.** 1992. Coamplified positive control detects inhibition of polymerase chain reactions. J. Clin. Microbiol. **30:**3185-3189.
11. **Dreyer, K., and H. Schulte-Holthausen.** 1991. Casein is a potent enhancer of restriction enzyme activity. Nucleic Acids Res. **19:**4295.
12. **Dyer, J.R., B.L. Gilliam, J.J. Eron Jr, L. Grosso, M.S. Cohen, and S.A. Fiscus.** 1996. Quantitation of human immunodeficiency virus type 1 RNA in cell free seminal plasma: comparison of NASBA ™ with Amplicor™ reverse transcription-PCR amplification and correlation with quantitative culture. J. Virological Meth. **60:**161-170.
13. **Fidler, H.M., G.A. Rook, N.M. Johnson, and J. McFadden.** 1993. Mycobacterium tuberculosis DNA in tissue affected by sarcoidosis. British Medical Journal **306:**546-549.
14. **Gerna,G., M. Furione, F. Baldanti, and A. Sarasini.** 1994. Comparative quantitation of human cytomegalovirus DNA in blood leucocytes and plasma of transplant and AIDS patients. J. Clin. Microbiol. **32:**2709-2717.
15. **Green, J., J.P. Norcott, D. Lewis, C. Arnold, and D.W.G. Brown.** 1993. Norwalk like viruses: Demonstration of genomic diversity by polymerase chain reaction. J. Clin. Microbiol. **31:**3007-3012.
16. **Hale, A.D., J. Green, andD.W.G. Brown.** 1996. Comparison of four RNA extraction methods for the detection of small round viruses in faecal specimens. J. of Virological Methods **57:** 195-201.
17. **Handt, O., M. Höss, M. Krings, and S. Pääbo.** 1994. Ancient DNA: methodological challenges. Experientia **50:**524-529.
18. **Ish-Horowicz, D., and J.F. Burke.** 1981. Rapid and efficient cosmid cloning. Nucleic Acids Res. **9:** 2989-2998.37.
19. **Kievits T., B. van Gemen, D. van Strijp, R. Schukkink, M. Dircks, H. Adriaanse, L. Malek, R. Sooknanan, and P. Lens.** 1991. NASBA isothermal enzymatic in vitro nucleic acid amplification optimized for the diagnosis of HIV-1 infection. J. Virol. Methods **35:**273-286.
20. **Kolk, A.H.J., A.R.J. Schuitema, S. Kuiper, J. van Leeuwen, P.M.W. Hermans, J.D.A. van Embden, and R.A. Hartskeerl.** 1992. Detection of Mycobacterium tuberculosis in clinical samples by using polymerase chain reaction and a nonradioactive detection system. J. Clin. Microbiol. **30:**2567-2575.
21. **Koopmans M., E.J. Snijder, and M.C. Horzinek.** 1991. cDNA probes for the diagnosis of bovine torovirus (breda virus) infection. J. Clin. Microbiol. **29:**493-497.
22. **Kreader, C.A.** 1996. Relief of amplification inhibition in PCR with bovine serum albumin or T4 gene 32 protein. Appl. Environ. Microbiol. **62:**1102-1106.
23. **Laemmli, U.K.** 1970. Cleavage of structural proteins during the assembly of the head of bacteriophage T4. Nature **227:**680-685.
24. **McCaustland, K.A., S. Bi, M.A. Purdy, and D.W. Bradley.** 1991. Application of two RNA extraction methods prior to amplification of hepatitis E virus nucleic acid by the polymerase chain reaction. J. Virol. Methods **35:**331-342.
25. **Merien, F., P. Perolat, E. Mancel, D. Persan, and G. Baranton.** 1993. Detection of Leptospira DNA by polymerase chain reaction in aqueous humour of a patient with unilateral uveitis. J. Infect. Diseases **168:**1335-1336.
26. **Morsica, G., N. Novati, and A. Lazzarin.** 1995. Detection of Hepatitis C virus RNA by methods using phenol-chloroform or silica particles. J. Clin. Microbiol. **33**:2522-2523.
27. **Noordhoek, G.T., E.C. Wolters, M.E.J. de Jonge, and J.D.A van Embden.** 1991. Detection by polymerase chain reaction of Treponema pallidum DNA in cerebrospinal fluid from neurosyphilis patients before and after antibiotic treatment. J. Clin. Microbiol. **29:**1976-1984.
28. **O'Sullivan, M.G., D.C. Anderson, J.D. Fikes, F.T. Bain, C.S. Carlson, S.W. Green, N.S. Young, and K.E. Brown.** 1994. Identification of a novel simian Parvovirus in Cynomolgus monkeys with severe anaemia. J. Clin. Investigation **93:**1571-1576.
29. **Parvaz, P., E. Guichard, P. Chevallier, J. Ritter, C. Trepo, and M. Sepetjan.** 1994. Hepatitis C: description of a highly sensitive method for clinical detection of viral RNA. J. Virol. Methods **47:**83-94.
30. **Rasmussen, L.K., P. Hojrup, and T.E. Petersen.** 1994. Disulphide arrangement in bovine caseins: localization of intrachain disulphide bridges in monomers of kappa- and alpha s2-casein from bovine milk. J. Dairy Res. **61:**485-493.
31. **Revello, M.G., F. Baldanti, A. Sarasini, D. Zella, M. Zavattoni, and G. Gerna.** 1997. Quantitation of herpes simplex virus DNA in cerebrospinal fluid of patients with herpes simplex encephalitis by the polymerase chain reaction. Clinical and Diagnostic Virology **7:**183-191.
32. **Revello, M.G., F. Baldanti, A. Sarasini, M. Zavattoni, M.Torsellini, and G. Gerna.** 1997. Prenatal diagnosis of rubella virus infection by direct detection and semiquantitation of viral RNA in clinical samples by reverse transcriptase-PCR. J. Clin. Microbiol. **35:**708-713.
33. **Romano, J.W., K.G. Williams, R.N. Shurtliff, C. Ginocchio, and M. Kaplan.** 1997. NASBA technology: isothermal RNA amplification in qualitative and quantitative diagnostics. Immunological Invest. **26:**15-28.
34. **Saboor, S.A., N.M. Johnson, and J. McFadden.** 1993. Detection of mycobacterial DNA in sarcoidosis and tuberculosis with polymerase chain reaction. Lancet **339:**1012-1015.
35. **Saiki, R.K., D.H. Gelfland, S. Stoffel, S.J. Scharf, R. Higuchi, G.T. Horn, K.B. Mullis, and H.A. Erlich.** 1988. Primer-directed enzymatic amplification of DNA with a thermostable DNA polymerase. Science **239:** 487-491.
36. **Secchiero, P., D.R. Carrigan, Y. Asano, L. Benedettti, R.W. Crowley, A.L. Komaroff, R.C. Gallo and P. Lusso.** 1995. Detection of human herpesvirus 6 in plasma of children with primary infection and immunosuppressed patients by polymerase chain reaction. J. Infect. Dis. **171:**273-280.
37. **Schirm, J., L.A.B. Oostendorp, and J.G. Mulder.** 1995. Comparison of Amplicor, in-house PCR, and conventional culture for detection of mycobacterium tuberculosis in clinical samples. J. Clin. Microbiol. **33:**3221-4224.
38. **Schuurman R., B. van Steenis, A. van Strien, J. van der Noordaa, and C. Sol.** 1991. Frequent detection of bovine polyomavirus in commercial batches of calf serum by using the polymerase chain reaction. J. General Virol. **72:**2739-2745.
39. **Shafer, R.W., D.J. Levee, M.A. Winters, K.L. Richmond, D. Huang, and T. C. Merigan.** 1997. Comparison of QIAamp HCV kit spin columns, silica beads, and phenol chloroform for recovering human immunodeficiency virus type 1 RNA from plasma. J. Clin. Microbiol. **35:**520-522.
40. **Swaisgood, H.E.** 1992. Chemistry of the caseins. In: Advanced Dairy Chemistry (Edited by Fox, P.F.), **1:**63-110, Elsevier Applied Science, London.
41. **Tieben, L.M., J. Terschegget, R.P. Minnaar, J.N.B. Bavinck, R.J.M. Berkhout, B.J. Vermeer, M.F. Jebbink, and H.L. Smits.** 1993. Detection of cutaneous and genital HPV types in clinical samples by PCR using consensus primers. J. Virol. Methods **42:**265-280.
42. **Todd, D., K.A. Mahwhinney, and M.S. McNulty.** 1992. Detection and differentiation of chicken anaemia virus isolates by using the polymerase chain reaction. J. Clin. Microbiol. **30:**1661-1666.
43. **Vandamme, A., S. van Dooren, W. Kok, P. Goubau, K. Fransen, T. Kievits, J. Schmit, E. de Clercq, and J. Desmyter.** 1995. Detection of HIV-1 RNA in plasma and serum samples using the NASBA amplification system compared to RNA-PCR. J. Virological Meth. **52:**121-131.
44. **Van der Hoek, L., R. Boom, J. Goudsmit, F. Snijders, and C.J.A. Sol.** 1995. Isolation of human immunodeficiency virus type 1 (HIV-1) RNA from feces by a simple method and difference between subpopulations in feces and serum. J. Clin. Microbiol. **33:**581-588.
45. **Van Gemen, B., T. Kievits, R. Schukkink, D. van Strijp, L.T. Malek, R. Sooknanan, H.G. Huisman, and P. Lens.** 1993. Quantification of HIV-RNA in plasma using NASBA^{™} during HIV-1 primary infection. J. Virol. Methods **43:** 177-188.
46. **Van Gemen, B., T. Kievits, P. Nara, H.G. Huisman, S. Jurriaans. J. Goudsmit, and P. Lens.** 1993. Qualitative and quantitative detection of HIV-1 RNA by nucleic acid sequence-based amplification. AIDS **7:**S107-S110.
47. **Vernazza, P.L., J.R. Dyer, S.A. Fiscus, J.J. Eron, and M.S. Cohen.** 1997. HIV-1 viral load in blood, semen and saliva. AIDS **11:**1058-1059.
48. **Wall, S., Z.M. Kunze, S. Saboor, J. Soufleri, P. Seechurn, R. Chiodini, and J.J. McFadden.** 1993. Identification of spheroplast-like agents isolated from tissues of patients with Crohn's disease and control tissues by polymerase chain reaction. J. Clin. Microbiol. **31:**1241-1245.
49. **Zipeto, D., F. Baldanti, D. Zella, M. Furione, A. Cavicchini, G. Milanesi, and G. Gerna.** 1993. Quantification of human cytomegalovirus DNA in peripheral blood polymorphonuclear leukocytes of immunocompromised patients by the polymerase chain reaction. J. Virological Methods **44:**45-56.
50. **Zoll G.J., W.J.G. Melchers, H. Kopecka, G. Jambroes, H.J.A van der Poel, and J.M.D. Galama.** 1992. General primer-mediated polymerase chain reaction for detection of enterovirus: application for diagnostic routine and persistent infections. J. Clin. Microbiol. **30:**160-165. All references mentioned herein are incorporated herein by reference.

## Claims

1. A method for isolating and identifying a target nucleic acid from samples containing biological material, comprising lysing said biological material with a lysis buffer and processing a target nucleic acid suspected to be present in said sample, whereby casein or a functional equivalent thereof is added to avoid inhibition of nucleic acid processing enzymes.

2. A method according to claim 1, whereby casein or its functional equivalent is added to said lysis buffer.

3. A method according to claim 1 or 2, whereby said processing includes amplification of target nucleic acid.

4. A method according to anyone of the aforegoing claims wherein said processing includes restriction and/or ligation of target nucleic acids.

5. A method according to any one of the aforegoing claims whereby said target nucleic acids are double strand nucleic acids, in particular double stranded DNA.

6. A method according to anyone of the aforegoing claims whereby the target nucleic acids are present in the sample at the most at levels of 50.000 molecules per 200 µl of sample.

7. A method according to claim 6, whereby the target nucleic acids are present in the sample at the most at levels of 20.000 molecules per 200 µl of sample.

8. A method according to anyone of the aforegoing claims whereby the sample is an aqeous body fluid, particularly derived from urine or cerebrospinal fluid or feces.

9. A method according to anyone of the aforegoing claims whereby the target nucleic acid originates from a pathogen, in particular a virus.

10. A method according to anyone of the aforegoing claims whereby casein is bovine alpha casein.

11. A method according to anyone of the aforegoing claims whereby the sample is contacted with silica particles during and/or after lysing.

12. A method according to claim 11 whereby the silica particles are present in the lysis buffer.

13. A method according to anyone of the aforegoing claims whereby the lysis buffer comprises a chaotropic agent, in particular guanidiniumthiocyanate.
